# EUROPEAN PATENT APPLICATION

(11) **EP 4 458 762 A1**
(43) Date of publication of application: **06.11.2024**
(21) Application number: 23315165.3
(22) Date of filing: 05.05.2023
(51) Int. Cl.: C01B 3/02, C01B 3/04, C01B 13/02

(54) **METHOD FOR CERTIFYING ORIGIN OF A HYDROGEN-BASED FLOW OR OF AN OXYGEN-BASED FLOW**

(71) Applicant: TOTALENERGIES ONETECH, 92400 Courbevoie (FR)
(72) Inventor: Dubouis, Nicolas, 92078 PARIS LA DEFENSE (FR); Ayme-Perrot, David, 92078 PARIS LA DEFENSE (FR)
(74) Representative: Lavoix

(57) **Abstract**

The invention relates to a method for certifying origin of a hydrogen-based flow or of an oxygen-based flow, comprising the following steps:
(i) Generating a produced flow of dihydrogen (23) and/or dihydrogen derivatives, respectively a produced flow of dioxygen, from an initial flow (22);
(ii) Tagging at least one of the initial flow (22) and/or of the produced flow (23) by adding a determined amount of stable isotope chosen between deuterium, tritium, ¹⁸O and ¹⁷O;
(iii) Measuring the concentration of said isotope in the initial flow (22) and/or the produced flow (23) to obtain a primary isotope ratio ;
(iv) Associating said primary isotope ratio with a unique identifier to form identification information and storing the identification information in a distant server.

## Description

### FIELD OF THE INVENTION

The present invention concerns the field of clean energy, and more particularly a method for authenticating the origin of a production of clean hydrogen or oxygen. The present invention particularly relates to a method for certifying the origin of a hydrogen-based or oxygen-based flow, as well as a computer program product and a system for carrying out such method.

### BACKGROUND OF THE INVENTION

Hydrogen can be produced from various energy sources, and the cost, amount, and energy-footprint of hydrogen vary, depending on production methods.

The type of method used for forming the hydrogen greatly affects the environmental value (CO2 emission in production, transportation, and the like) and economic value of hydrogen.

In this connection, a certification project have been launched in Europe in efforts for establishing a hydrogen trading market. In this project, the environmental value of hydrogen is defined in three categories, that is, Green hydrogen, Low Carbon hydrogen, and Gray hydrogen, based on CO2 emission intensity. Gray hydrogen is, for example, natural gas reformed hydrogen, which is produced from a fossil fuel as a raw material. Its CO2 emission intensity is therefore high, because of which its environmental value is evaluated to be low. Green hydrogen (e.g., hydrogen produced by electrolyzing water using electricity generated by renewable power generation, such as wind power generation or solar power generation) and Low Carbon hydrogen (e.g., hydrogen produced by electrolyzing water using electricity from power generation systems), on the other hand, show low CO2 emission intensity, and are therefore evaluated to be low in environmental value.

The demand for green hydrogen or low-carbon hydrogen in the industry is increasing worldwide. However, from a molecular point of view, a green dihydrogen molecule or low carbon dihydrogen molecule are the same as gray dihydrogen molecule.

Certification of origin based on digital methods are employed for tracking green electricity and potentially green hydrogen and downstream products, for example by issuing certificate following a regular audit of the production chain, the certificate being handed to the hydrogen purchaser at the time of the hydrogen purchase.

However, these techniques are not entirely satisfying, and they need improvement to guarantee transparency and reliability in the certification of origin of hydrogen, hydrogen derivatives or other gas such as oxygen.

### SUMMARY OF THE INVENTION

One aim of the present invention is to improve the existing techniques and to make it possible to authenticate the origin of hydrogen-based flow or of an oxygen-based flow in a simple and reliable way, without altering the quality of the hydrogen- or oxygen-based flow.

To that end, the present invention relates to a method for certifying origin of a hydrogen-based flow or of an oxygen-based flow, comprising the following steps:
(i) Generating, notably by water electrolysis or natural gas reforming, a produced flow of dihydrogen and/or dihydrogen derivatives, respectively a produced flow of dioxygen, from an initial flow;
(ii) Tagging at least one of the initial flow and/or of the produced flow by adding a determined amount of stable isotope chosen between deuterium, tritium, ¹⁸O and ¹⁷O;
(iii) Measuring the concentration of said isotope in the initial flow and/or the produced flow to obtain a primary isotope ratio;
(iv) Associating said primary isotope ratio with a unique identifier to form identification information and storing the identification information in a distant server.

The method according to the invention allows a reliable certification of the origin of an hydrogen-based flow or an oxygen-based flow, via the analysis of ratios of stable isotopes, combined with the generation of reliable identification information.

The unique identifier allows traceability of the hydrogen-based flow or oxygen-based flow, making it possible to determine its origin,

Stable isotopes are not considered impurities and they do not affect negatively the quality of the hydrogen-based flow or oxygen-based flow, making these flows suitable for applications, such as in fuel cells.

Physical/chemical tracing of these molecules flow is particularly helpful for decentralized production and distribution, and favors control by authorities of the origin of the molecules.

The method according to the invention may comprise one or more of the following features, taken alone or in any technically feasible combination:
- the method further comprises the steps of (v) comparing the identification information with a request for authenticating, said request for authenticating comprising a measured isotope ratio of a hydrogen-based flow to be authenticated, respectively an oxygen-based flow to be authenticated; and (vi) based on the comparison, generating an authentication confirmation if the identification information matches the request for authenticating.
- the measured isotope ratio of the flow to be authenticated is obtained by spectroscopy, preferably by high-resolution mass spectrometry.
- the method further comprises a step of storing and transporting the produced flow prior to the step of comparing the identification information with a request for authenticating.
- the produced flow of dihydrogen and/or dihydrogen derivatives is generated by water electrolysis of a water flow, the tagging step comprising (iia) injecting heavy water D₂O and/or tritiated water T₂O into the water flow.
- the tagging step comprises (iib) injecting a gas chosen between D₂ gas and T₂ gas into the produced flow of dihydrogen.
- the step of generating the produced flow of dihydrogen derivatives comprises a step of generating a flow of dihydrogen and a step of converting said flow of dihydrogen into a produced hydrogen-based flow by chemical reaction with a reacting agent from a reacting flow, said produced hydrogen-based flow comprising H₂ derivatives, preferably chosen between NH₂, CH₄ or CH₂OH, or methylcyclohexane.
- the produced flow of oxygen is generated by water electrolysis of a water flow, the tagging step comprising (iia) injecting ¹⁸O-enriched water (H₂¹⁸O) into the water flow or injecting ¹⁷O-enriched water (H₂¹⁷O) into the water flow.
- the tagging step comprises (iib) injecting Oxygen-¹⁸O₂ into the produced flow of oxygen or injecting Oxygen-¹⁷O₂ into the produced flow of oxygen.
- the unique identifier is obtained by computing the primary isotope ratio with a partition coefficient specific to the generating step, the unique identifier being preferably defined by an electronic computer, this electronic computer comprising a model having in memory the partition coefficient specific to the generating step.
- the method further comprises a step of emitting a certificate comprising the identification information and sending said certificate to an electronic receiving device.
- the certificate is an electronic certificate comprising a unique token, said token being associated to a purchased number ranging from 0 to 1 (excluded) corresponding to the unique identifier, the step of associating the token to a purchased number being preferably done by an algorithm.
- the certificate is associated with a validity period, the quantity of added isotope being adjusted so that the produced flow of dihydrogen and/or dihydrogen derivatives, respectively of the produced flow of dioxygen, generated during said validity period has the same unique identifier.

The invention also relates to a computer program product comprising software instructions which, when executed by a processor, implement a method as described above.

The invention also relates to a system for certifying origin of a hydrogen-based flow and/or of an oxygen-based flow, the system being configured to carry out the method as described above, the system comprising:
- a tagging device for tagging at least one of the initial flow and/or of the produced flow by adding a determined amount of isotope;
- a measuring device for measuring the concentration of said isotope in the initial flow and/or the produced flow to obtain a primary isotope ratio;
- an identification device for associating said primary isotope ratio with a unique identifier to form identification information and storing the identification information in a distant server.

### BRIEF DESCRIPTION OF THE DRAWINGS

The invention and its advantages will be better understood upon reading the following description, which is given solely by way of non-limiting example and which is made with reference to the appended drawings, in which:
- Figure 1 is a schematic diagram showing a system for certifying origin of a hydrogen-based flow according to a first embodiment of the invention.
- Figure 2 is a flowchart of a method for certifying origin of a purchased hydrogen-based flow, the method being performed by the system of Figure 1.
- Figure 3 is a flowchart of a variant of the method of Figure 2.
- Figure 4 is a schematic diagram showing a system for certifying origin of a hydrogen-based flow according to a second embodiment of the invention.
- Figure 5 is a flowchart of a method for certifying origin of a purchased hydrogen-based flow, the method being performed by the system of Figure 4.
- Figure 6 is a schematic diagram showing a system for certifying origin of a oxygen-based flow according to a third embodiment of the invention.
- Figure 7 is a flowchart of a method for certifying origin of a purchased oxygen-based flow, the method being performed by the system of Figure 6.

Before describing the invention, it is to be understood that it is not limited to the details of construction or process steps set forth in the following description. It will be apparent to those skilled in the art having the benefit of the present disclosure that invention is capable of other embodiments and of being practiced or being carried out in various ways.

in the following description, the term "hydrogen-based flow" is used in reference to a flow of dihydrogen or a flow of dihydrogen derivatives. Said dihydrogen derivatives are made from H₂ molecules that originate for example from water electrolysis, natural gas reforming or any other suitable processes and from other molecules, such as N₂, CO₂, CO, toluene, methyltoluene or any other hydrogen carriers

In the following description, the term "oxygen-based flow" is used in reference to a flow of dioxygen.

In the following description, unless indicated otherwise, the ratio of A/B, also called A/B ratio, is to be understood as the ratio of the mass concentration of element A by the mass concentration of element B.

As used herein, the term "distant server" may refer to one or several computers able to provide a distant service such for one or several certification systems. The distant service can be accessible by a user via a terminal. In some embodiments, the distant server is able to store electronic certificates.

As used herein, the term "unique identifier" may refer to a number or a series of alphanumeric characters or any other form of identification, making it possible to identify each initial and reacting flows, preferably in a unique way.

### FIRST EMBODIMENT OF THE INVENTION

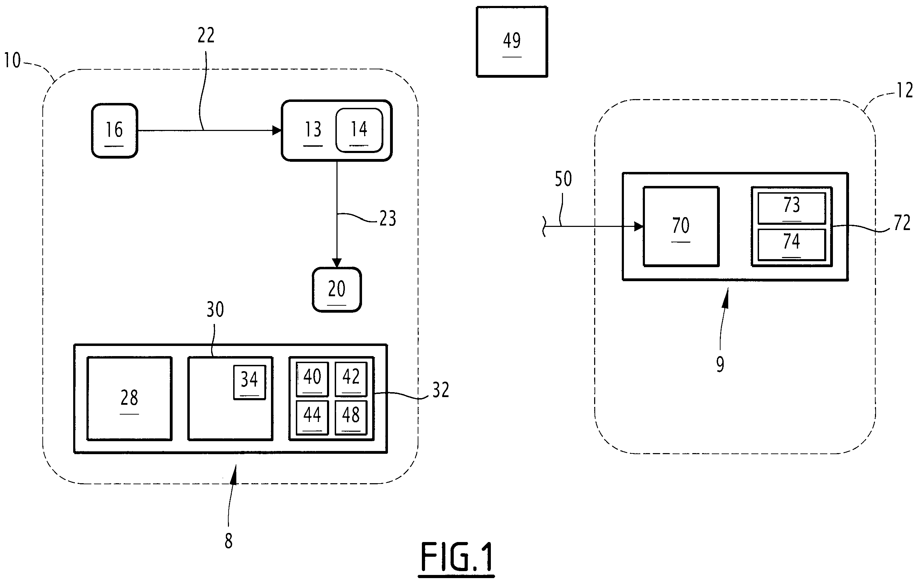

Referring to Figure 1, a system 8 for certifying origin of a hydrogen-based flow, also called certification system 8, according to a first embodiment of the invention will be first described.

Particularly, the system 8 is configured to be implemented in an installation 10 for producing a hydrogen-based flow, also called producing installation 10.

The hydrogen-based flow is intended to be transported to and received by a second installation 12, called purchasing installation 12. Said purchasing installation 12 may be for example a hydrogen refuelling station, a refinery, a dehydrogenation unit, a Haber-Bosch plant or any other chemical plants.

As it will be described later, the purchasing installation 12 comprises a system 9 for verifying the origin of a hydrogen-based flow, also called verification system 9.

The producing installation 10 comprises a generation unit 13 configured to produce a produced flow of dihydrogen 23 from an initial flow 22.

For example, in the embodiment shown on Figure 1, the installation 10 is an electrochemical installation, notably for the generation of dihydrogen and of dioxygen from water electrolysis.

The generation unit 13 comprises an electrolysis unit 14 adapted for producing a dihydrogen flow 23 by water electrolysis and a balance of plant connected to the electrolysis unit 14, the balance of plant comprising various fluid handling components to manage the incoming fluids (in particular steam, electricity, air/nitrogen) and the outgoing fluids (hydrogen and oxygen).

The installation 10 comprises an electrolyte reservoir 16 fluidly connected to the electrolysis unit 14 and configured to supply the electrolysis unit 14 with electrolyte, which is the initial flow. The electrolyte reservoir 16 is for example a tank, a pipe system or any other enclosure suitable for the electrolyte.

Advantageously, the electrolyte is water obtained by seawater purification. The electrolyte reservoir 16 is for example connected to a desalinization assembly (not shown).

The installation 10 further comprises a storage unit 20.

The storage unit 20 is configured to store the produced dihydrogen flow 23, before its delivery to the purchasing installation 12. For example, the storage unit 20 comprises compression systems, liquefaction systems and/or transformation systems to compress, liquefy or transform the dihydrogen in any other molecule before its delivery to facilitate its transport.

The system 8 comprises a tagging device 28, a measuring device 30 and an identification device 32.

The tagging device 28 is configured to tag at least one of the initial flow 22 and/or of the produced flow 23 by adding a determined amount of stable isotope.

According to the first embodiment of the invention, the stable isotope is chosen between deuterium (D) and tritium (T).

For example, in the embodiment shown on Figure 1, the tagging device 28 is configured to tag the initial flow 22 (which is water) by injecting heavy water D₂O or tritiated water (HTO or T₂O) into the initial flow 22.

The measuring device 30 is configured to measure D/H or T/H ratios in samples of the initial flow 22 to obtain a primary isotope ratio.

Preferably, the measuring device 30 comprises a communication module 34 suitable to communicate the obtained measurements to the identification device 32.

For example, the measuring device 30 comprises a gas chromatographer, preferably a gas chromatography-isotope ratio-mass spectrometer.

The tagging device 28 and the measuring device 30 may be part of a same device.

The identification device 32 is configured to receive the primary isotope ratios communicated by the measuring device 30 and to process them in order to form identification information.

According to the first embodiment, the identification device 32 comprises an association module 40, a computation module 42, a certificate emission module 44 and a transmission module 48.

The association module 40 is configured to receive primary isotope ratios from the communication module 34 of the measuring device 30. In complement or in a variant, the association module 40 is configured to receive isotope ratios entered manually, for example thanks to a manual input device.

The association module 40 is configured to associate each primary isotope ratio with a unique identifier, in order to form identification information.

The unique identifier associated with the initial flow 22 includes information about the origin of the hydrogen, in particular the hydrogen type and/or the hydrogen production method.

Hydrogen type is information indicating types of hydrogen, such as Gray hydrogen, Low Carbon hydrogen, and Green hydrogen.

Hydrogen production method is information indicating the method by which hydrogen is produced.

Using the unique identifier, the association module 40 is able to form identification information for the initial flow.

The computation module 42 is configured to compute the identification information with partition coefficient specific to the process implemented by the generation unit 13, as it will be explained later. The computation module 42 is configured to generate an expected isotope ratio based on said computation.

The certificate emission module 44 is configured to issue a certificate comprising the expected ratio. Advantageously, the certificate is an electronic certificate certifying the origin of the produced hydrogen-based flow. The certificate may contain additional information, such as the date of the certificate issuance, the date of production of the hydrogen flow, the origin of the energy used to produce the hydrogen flow, the name of the producer of the hydrogen-based flow or the GPS coordinates of the production site.

In a preferred embodiment, the certificate comprises a unique token, associated to a purchased number ranging from 0 to 1 (excluded) corresponding to the expected isotope ratio. The association of the token to a purchased number is preferably done by an algorithm.

Advantageously, the information contained in the certificate is encrypted.

The transmission module 48 is configured to send the certificate to a distant server 49. For this purpose, the transmission module 48 is connected to the distant server 49 using for example a global communication network such as Internet.

The identification device 32 is typically an electronic computer provided with a memory and a microprocessor associated to said memory. The association module 40, the computation module 42, the certificate emission module 44 and the transmission module 48 are each realized in the form of a software, or a software brick, executable by the microprocessor.

Advantageously, the memory is able to store a model comprising the partition coefficients specific to the processes implemented by the generation unit 13 and the conversion unit 18. The processor is able to execute said model.

In a variant, the association module 40, the computation module 42, the certificate emission module 44 and the transmission module 48 are each realized in the form of a programmable logic component, such as an FPGA (Field Programmable Gate Array), or in the form of a dedicated integrated circuit, such as an ASIC (Application Specific Integrated Circuit).

When the identification device 32 is realized in the form of one or more software programs, i.e., in the form of a computer program, it is further adapted to be recorded on a computer-readable medium, not shown. The computer-readable medium is, for example, a medium capable of storing electronic instructions and of being coupled to a bus of a computer system. For example, the readable medium is an optical disk, a magneto-optical disk, a ROM memory, a RAM memory, any type of non-volatile memory (e.g. EPROM, EEPROM, FLASH, NVRAM), a magnetic card or an optical card. A computer program with software instructions is stored on the readable medium.

As mentioned above, the system 9 for verifying the origin of a hydrogen-based flow is located in the purchasing installation 12.

The system 9 comprises a measuring device 70 for measuring isotope ratios in a purchased hydrogen-based flow 50 and an electronic-receiving device 72.

The electronic-receiving device 72 is configured to access to a certificate stored in the distant server 49.

Preferably, the electronic-receiving device 72 comprises a reception module 73 configured to receive the certificate and a comparison module 74 configured to compare the expected ratio registered in said certificate and the measured ratio obtained by the measuring device 70.

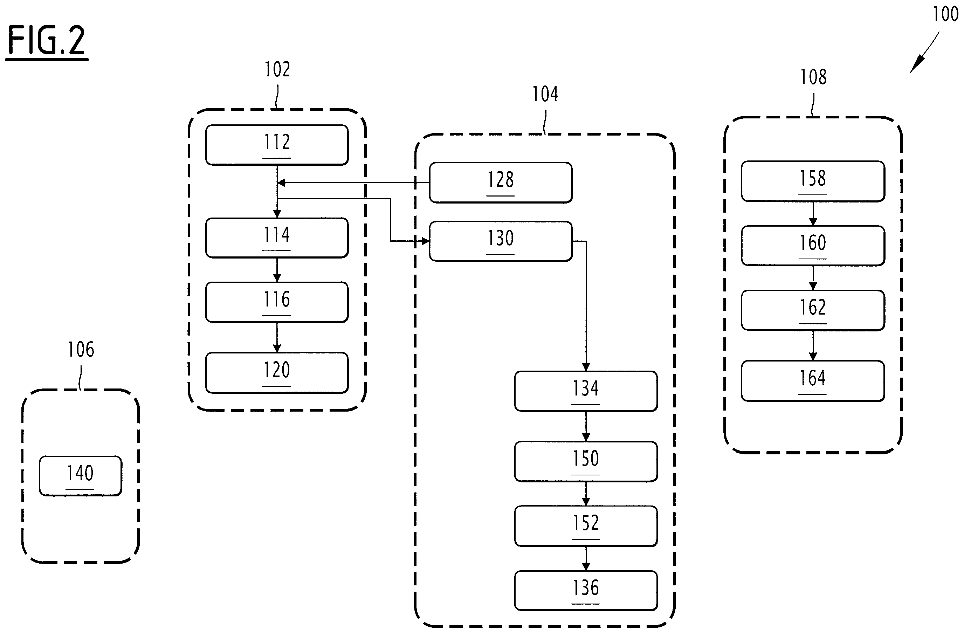

A method for certifying origin of a hydrogen-based flow, called also method 100, will now be explained in reference to Figure 2 presenting a flowchart of its steps. The method 100 is performed by the system 8 according to the first embodiment of the invention.

The method 100 comprises a production phase 102 and a certification phase 104. Advantageously, the method 100 further comprises a preliminary calibration phase 106 and a final authentication phase 108. Typically, the calibration, the production and the certification phases 106, 102, 104 are carried out by in the producing installation 10 and the authentication phase 108 is carried out in the purchasing installation 12.

The production phase 102 is typically carried out in the producing installation 10.

Initially, an initial flow 22 is provided to the generation unit 13 in an initial supplying step 112.

In the embodiment of Figure 2, the electrolyte is supplied into the electrolysis unit 14 of the installation 10.

A produced flow of dihydrogen 23 is then generated in the electrolysis unit 14 during a generating step 114, by water electrolysis. As known per se, electrolysis of water is the decomposition of water (H₂O) into dioxygen (O₂) and dihydrogen gas (H₂) due to an electric current being passed through the water.

The produced flow of dihydrogen 23 is then collected during a collecting step 116.

Then, during a storing step 120, the produced flow of dihydrogen 23 is stored in the storage unit 20, before being shipped to the purchasing installation 12. For example, the produced flow of dihydrogen 23 may be compressed or liquefied during step 120 and is typically stored in tanks or underground reservoirs.

The certification phase 104 is carried out at least partly simultaneously to the production phase 102.

The certification phase 104 comprises a tagging step 128, a measuring step 130, an associating step 134 and an information storing step 136.

The tagging step 128 and the measuring step 130 may be done simultaneously or successively.

Prior to the generating step 114, a determined amount of stable isotope chosen between deuterium and tritium is added in the initial flow 22 during the tagging step 128.

According to the first embodiment, heavy water D₂O and/or tritiated water T₂O is injected into the water flow 22.

The measuring step 130 is carried out just after the tagging step 128. During this step, the D/H or T/H ratio is measured by the measuring device 30 in a sample of the tagged initial flow.

After tagging, the primary isotope ratio D/H or T/H is typically comprised between 0%wt (excluded) and 10.0%wt.

The isotope ratio measured during the measuring step 130 is called primary isotope ratio.

Advantageously, the measurements during step 130 are carried out by gas chromatography, notably by gas chromatography-isotope ratio-mass spectrometry.

Then, the association module 40 of the identification device 32 associates the primary isotope ratio with a unique identifier during the associating step 134. As explained above, the association module 40 can determine the isotope ratios using for example data transmitted by the communication module 34 of the measuring device 30 or by manual input.

During the associating step 134, the D/H or T/H ratio of the initial flow 22 of water is associated with a unique identifier to form hydrogen-related identification information. Said identification information is then stored in the distant server 49 during the information storing step 136.

The association during step 134 is carried out automatically by the association module 40.

According to the first embodiment, the certification phase 104 further comprises a computation step 150 and a certificate emission step 152. Said steps 150, 152 are carried out between the associating step 134 and the information storing step 136.

During the computation step 150, the identification information is computed with the partition coefficient specific to the generating step 114 to obtain an expected isotope ratio based on said computation

Said partition coefficient has preferably been previously obtained during the preliminary calibration phase 106, which will be detailed later.

Then, during the certificate emission step 152, a certificate comprising the expected isotope ratio is emitted by the certificate emission module 44.

The certificate is preferably an electronic certificate.

The certificate is then transmitted to and made available on the distant server 49, for example using blockchain technology. For this purpose, the transmission module 48 preferably uses a secure channel of the TLS type, such as an HTTPS channel which is encrypted and authenticated.

The partition coefficient specific to the generation step 114 is acquired during the preliminary calibration phase 106.

The preliminary calibration phase 106 comprises a step 140 of acquiring the partition coefficient specific to the generating step 114.

The partition coefficient is correlated to the variation of the stable isotope during the generating step 114.

During step 140, known quantities of heavy water D₂O or tritiated water H₂O are added into a set of water samples to carry out a calibration, so that the ratio of D/H or T/H in the samples varies from 0% to 100% (molar), preferably from 0% to 10% (molar).

For each sample, a dihydrogen flow sample is generated by water electrolysis using the generation device 13 with the same parameters than the one used during the generating step 114.

The D/H, respectively T/H, ratio of each dihydrogen flow sample is then measured, for example by spectroscopy, preferably by high resolution mass spectrometry.

The isotopic partition coefficient specific to the generating step 114 is thus obtained based on the variation of the stable isotope ratio during said step 114. Advantageously, said isotopic partition coefficient is then recorded in the model housed in the memory of the identification device 32.

The final authentication phase 108 is performed when one is intending to verify the origin of a hydrogen-based flow to be authenticated.

The final authentication phase 108 is preferably carried out in the purchasing installation 12.

First, a hydrogen-based flow 50, also called purchased hydrogen-based flow 50 or hydrogen-based flow to be authenticated 50 is obtained in an acquisition step 158.

During a final measuring step 160, isotope ratios of the purchased hydrogen-based flow 50 are measured, for example by using spectroscopy, preferably high-resolution mass spectrometry.

During step 160, the D/H, respectively the T/H, ratio is measured and a measured isotope ratio is thus obtained.

According to the first embodiment, the electronic-receiving device 72 then generates a request for receiving the certificate stored in the distant server 49. The certificate is received by the electronic-receiving device 72.

Advantageously, the distant server 49 is only accessible to authorized users.

Then, the measured ratio is compared with the expected isotope ratio during a comparing step 162.

If the expected isotope ratio matches the measured ratio, an authentication confirmation is then generated during an authenticating step 164.

If the expected isotope ratio does not match the measured ratio, no authentication confirmation is generated. Advantageously, a mismatch signal is then emitted during the authentication step 164.

By "matching", it is meant that the measured isotope ratio differs from the expected isotope ratio by no more than 10%, advantageously no more than 1.0%.

In a variant of the first embodiment, the tagging device 28 is configured to tag the produced dihydrogen flow 23 by injecting a gas chosen between D₂ gas and T₂ gas into the produced flow 23 of dihydrogen.

The measuring device 30 is configured to measure D/H or T/H ratios in samples of the produced dihydrogen flow 23 to obtain the primary isotope ratio.

The identification device 32 is configured to receive the primary isotope ratios communicated by the measuring device 30 and to process them in order to form identification information. According to this variant, the identification device 32 comprises an association module 40, a certificate emission module 44 and a transmission module 48. The identification device 32 may be deprived of a computation module.

The certificate emission module 44 is configured to issue a certificate comprising directly the primary isotope ratio. Advantageously, the certificate is an electronic certificate certifying the origin of the produced hydrogen-based flow. The certificate may contain additional information, such as the date of the certificate issuance, the date of production of the hydrogen-based flow, the name of the producer of the hydrogen-based flow or the GPS coordinates of the production site.

The method performed by the system according to the second variant of the first embodiment of the invention is similar to the method explained above, except the tagging step 128.

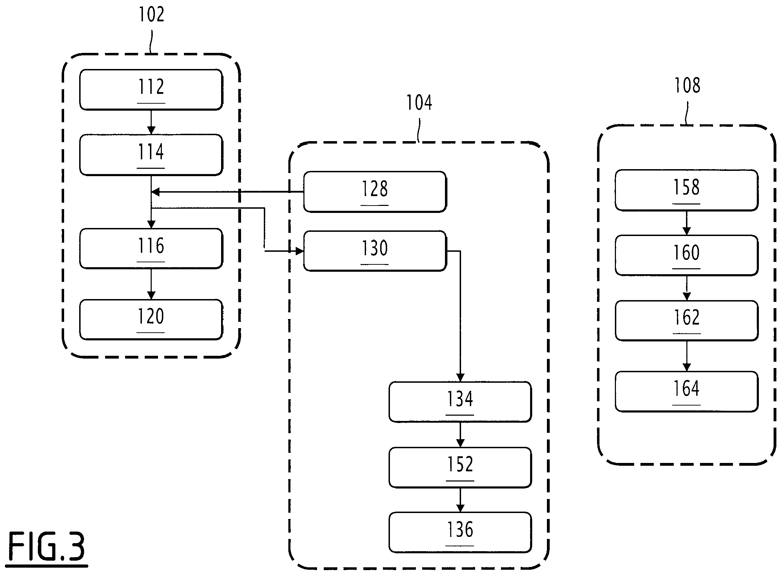

Figure 3 shows a flowchart of the steps of the method 100 performed by the system 8 according to said second variant.

The production phase 102 is similar to the first variant.

During the certification phase 104, the tagging step 128 is carried out after the generation step 114.

During the tagging step 128, a determined amount of stable isotope chosen between deuterium and tritium is added in the produced flow 23 of dihydrogen.

According to this second variant, a gas chosen between D2 gas and T2 gas is injected into the produced flow 23 of dihydrogen.

The measuring step 130 is carried out just after the tagging step 128. During this step, the D/H or T/H ratio is measured by the measuring device 30 in a sample of the tagged flow.

After tagging, the primary isotope ratio D/H or T/H is typically comprised between 0% (excluded) and 10.0% (molar ratio).

The isotope ratio measured during the measuring step 130 is called primary isotope ratio.

Advantageously, the measurements during step 130 are carried out by gas chromatography, notably by gas chromatography-isotope ratio-mass spectrometry.

Then, the association module 40 of the identification device 32 associates the primary isotope ratio with a unique identifier during the associating step 134. As explained above, the association module 40 can determine the isotope ratios using for example data transmitted by the communication module 34 of the measuring device 30 or by manual input.

During the associating step 134, the D/H or T/H ratio of the produced flow 23 is associated with a unique identifier to form hydrogen-related identification information. Said identification information is then stored in the distant server 49 during the information storing step 136.

The association during step 134 is carried out automatically by the association module 40.

According to the second variant of the first embodiment, the certification phase 104 further comprises a certificate emission step 152 which is carried out between the associating step 134 and the information storing step 136.

During the certificate emission step 152, a certificate comprising the primary isotope ratio is emitted by the certificate emission module 44.

### SECOND EMBODIMENT OF THE INVENTION

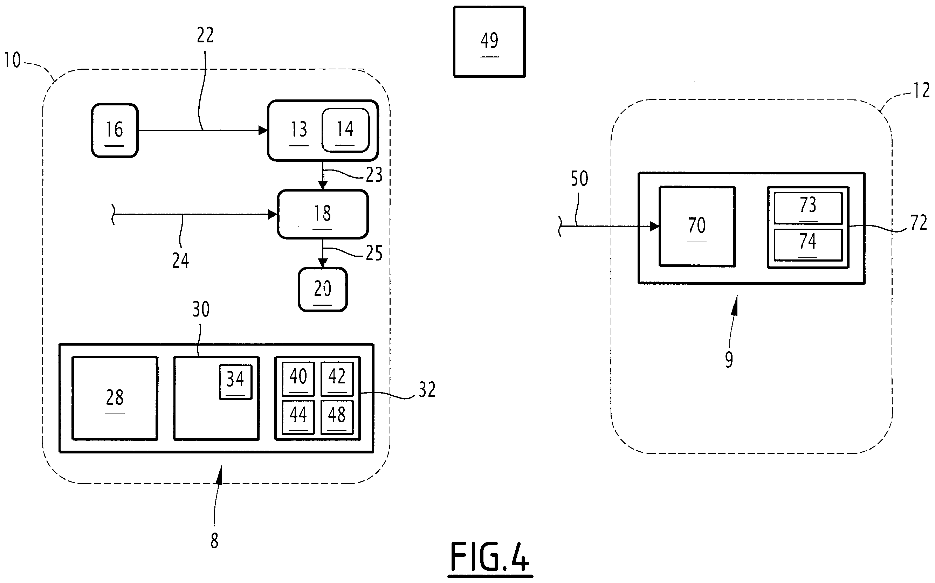

Figure 4 shows a system 8 for certifying origin of a hydrogen-based flow according to a second embodiment of the invention.

According to this embodiment, the system 8 comprises the same components as those explained above. Thus, the same numerical references will be used to designate these components.

Unlike the first embodiment, the hydrogen-based flow is here a flow of dihydrogen derivatives, typically chosen between NH₂, CH₄, CH₃OH and methycyclohexane

The installation 10 according to the second embodiment further comprises a conversion unit 18.

The conversion unit 18 is configured to convert the produced flow of dihydrogen 23 into a flow of dihydrogen derivatives 25, also called produced hydrogen-based flow 25, by chemical reaction with a reacting flow 24.

For example, the conversion unit 18 is a synthesis module for the production of methanol and/or methanol derivatives from hydrogen generated in the electrolysis unit 14 and CO₂ of a stream of CO₂-rich gas, said CO₂-rich gas coming for example from combustion of fuel. The reacting flow is here the stream of CO₂-rich gas.

In a variant, the conversion unit 18 is a methanation module for the production of methane CH₄ from hydrogen generated in the electrolysis unit 14 and CO₂ of a stream of CO₂-rich gas (Sabatier process). The reacting flow is here the stream of CO₂-rich gas.

In a variant, the conversion unit 18 is a synthesis module for the production of ammonia from hydrogen generated in the electrolysis unit 14 and diazote N₂ of a stream of N₂, by implementation of the Haber-Bosch catalytic process. The reacting flow is here the stream of N₂.

In a variant, the conversion unit 18 is a hydrogenation module for the production of methyltoluene from hydrogen generated in the electrolysis unit 14 and methylcyclohexane of a stream of CO₂-rich gas (Sabatier process). The reacting flow is here the stream of methylclohexane liquid.

The storage unit 20 is configured to store the produced hydrogen-based flow 25, before its delivery to the purchasing installation 12.

In the second embodiment, the computation module 42 is configured to compute each identification information with partition coefficients specific to the process implemented by the generation unit 13 and the conversion unit 18, as it will be explained later. The computation module 42 is configured to generate an expected isotope ratio based on said computation.

The certificate emission module 44 is configured to issue a certificate comprising said expected ratio.

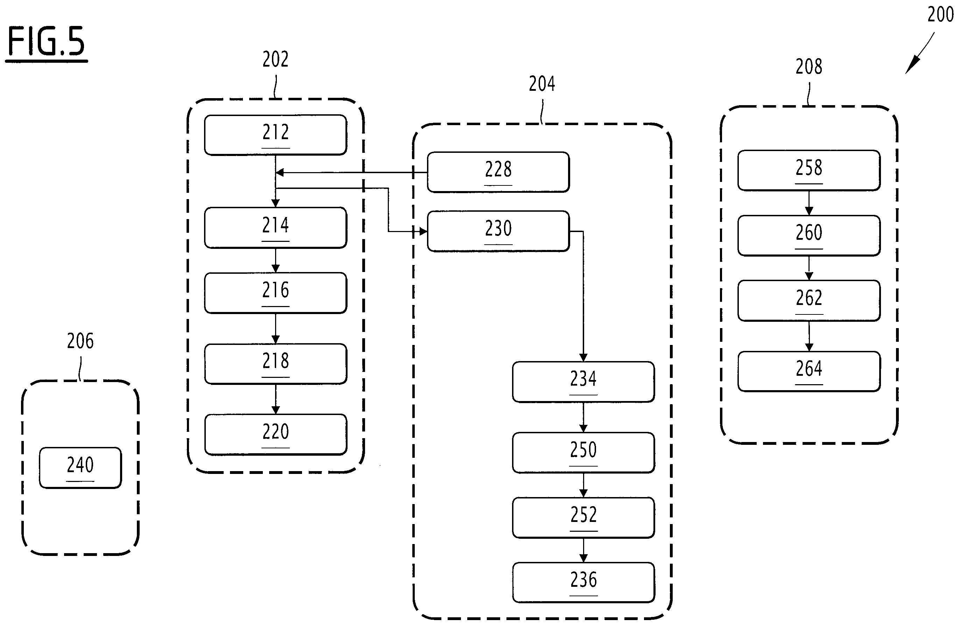

Figure 5 shows a flowchart of the steps of a method 200 for certifying origin of a hydrogen-based flow, performed by the system 8 according to the second embodiment of the invention.

The method 200 performed by the system according to the second embodiment of the invention is mostly similar to the method explained above. Thus, the same numerical references, only incremented by 100, will be used to designate the same phases or steps.

The production phase 202 is typically carried out in the producing installation 10 and comprises an initial supplying step 212, a generating step 214 and a collecting step 216 similar to those of the method of the first embodiment.

After the collecting step 216, the produced flow of dihydrogen 23 is then converted into a produced hydrogen-based flow 25 during a converting step 218.

For example, the produced flow of dihydrogen 23 is combined with a flow of nitrogen to produce ammonia (Haber-Bosch process). In a variant, the produced flow of dihydrogen 23 is mixed with a flow a carbon dioxide or carbon monoxide to form methane CH₄ or methanol CH₃OH.

Then, during the storing step 220, the produced flow of dihydrogen derivatives is stored in the storage unit 20, before being shipped to the purchasing installation 12. The produced flow of dihydrogen derivatives is typically stored in tanks or underground reservoirs.

The certification phase 204 is similar to the one of the first embodiment, except for the computation step 250.

According to the second embodiment, the identification information is computed with a partition coefficient specific to the production phase 202, to obtain an expected isotope ratio.

Said partition coefficient has preferably been previously obtained during the preliminary calibration phase 206, which will be detailed later.

The partition coefficient specific to the production phase 202 is acquired during the preliminary calibration phase 206.

The preliminary calibration phase 206 comprises a step 242 of acquiring the partition coefficient specific to the production phase 202.

The partition coefficient is correlated to the variation of the stable isotope during the production phase 202.

According to the embodiment shown on Figure 5, in which the tagging step 228 consists in injecting heavy water D₂O and/or tritiated water T₂O into the water flow 22, the partition coefficient is correlated to the variation of the stable isotope during the generating step 214 and the converting step 218.

Known quantities of heavy water D₂O or tritiated water T₂O are added into a set of water samples to carry out a calibration, so that the ratio of D/H, respectively T/H, in the samples varies from 0% to 100%, preferably from 0% to 10%.

For each sample, a dihydrogen flow sample is generated by water electrolysis using the generation device 13 with the same parameters than the one used during the generating step 214.

Then, for each dihydrogen flow sample, a sample of dihydrogen derivative is generated using the conversion unit 18 with the same parameters than the one used during the converting step 218.

The D/H, respectively T/H, ratio of each dihydrogen derivative sample is then measured, for example by spectroscopy, preferably by high resolution mass spectrometry.

The isotopic partition coefficient specific to the production phase is thus obtained based on the variation of the stable isotope ratio during said steps 214, 218. Advantageously, said isotopic partition coefficient is then recorded in the model housed in the memory of the identification device 32.

In a variant, when the tagging step 228 consists in injecting a gas chosen between D₂ gas and T₂ gas into the produced flow of dihydrogen, the partition coefficient is correlated to the variation of the stable isotope during the converting step 218.

Known quantities of D2 or T2 gas are added into dihydrogen samples to carry out a calibration, so that the ratio of D/H, respectively T/H, in the samples varies from 0% to 100% (molar), preferably from 0% to 10% (molar).

For each dihydrogen flow sample, a sample of dihydrogen derivative is generated using the conversion unit 18 with the same parameters than the one used during the converting step 218.

The D/H, respectively T/H, ratio of each dihydrogen derivative sample is then measured, for example by spectroscopy, preferably by high resolution mass spectrometry.

The isotopic partition coefficient specific to the production phase is thus obtained based on the variation of the stable isotope ratio during said step 218. Advantageously, said isotopic partition coefficient is then recorded in the model housed in the memory of the identification device 32.

The final authentication phase 208 is then similar to the one of the first embodiment.

### THIRD EMBODIMENT OF THE INVENTION

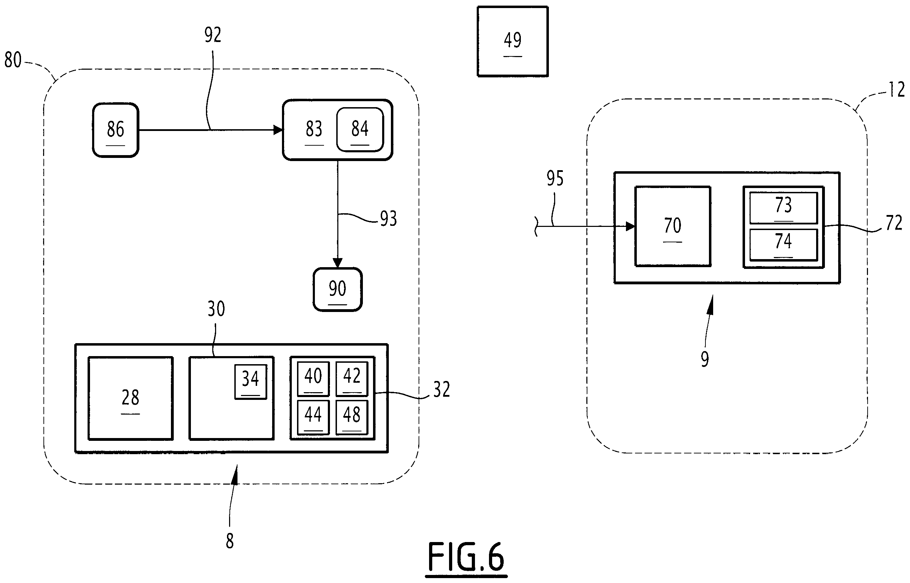

Figure 6 shows a system 8 for certifying origin of an oxygen-based flow according to a third embodiment of the invention.

According to this embodiment, the system 8 comprises the same components as those explained above. Thus, the same numerical references will be used to designate these components.

The system 8 according to the third embodiment is configured to be implemented in an installation 80 for producing an oxygen-based flow, also called producing installation 80.

The oxygen-based flow is intended to be transported to and received by a second installation 12, called purchasing installation 12. Said purchasing installation 12 may be for example an oxycombustion unit, a medical gas conditioning unit, a steel or metal manufacturing plant.

The producing installation 10 comprises a generation unit 83 configured to produce a produced flow of dioxygen 93 from an initial flow 92 coming from a reservoir 86.

For example, in the embodiment shown on Figure 1, the installation 80 is an electrochemical installation, notably for the generation of dihydrogen and of dioxygen from water electrolysis.

The generation unit 83 of the third embodiment is here similar to the generation unit 13 of the first and second embodiments and comprises a water electrolysis unit 84.

The installation 80 further comprises a storage unit 90 configured to store the produced dioxygen flow 93, before its delivery to the purchasing installation 12. For example, the storage unit 90 comprises compression systems and/or liquefaction systems to compress or liquefy the dioxygen before its delivery.

The system 8 comprises a tagging device 28, a measuring device 30 and an identification device 32.

The tagging device 28 is configured to tag at least one of the initial flow 92 and/or of the produced flow 93 by adding a determined amount of stable isotope.

According to the third embodiment of the invention, the stable isotope is ¹⁸O or ¹⁷O.

For example, in the embodiment shown on Figure 6, the tagging device 28 is configured to tag the initial flow 92 (which is water) by injecting ¹⁸O-enriched water (H₂¹⁸O) or ¹⁷O-enriched water (H₂¹⁷O) into the water flow

in a variant (not shown), the tagging device 28 is configured to tag the produced dioxygen flow 93 by injecting Oxygen-¹⁸O₂ into the produced flow of oxygen.

The measuring device 30 is configured to measure ¹⁸O/¹⁶O or ¹⁷O/¹⁶O ratios in samples of the initial flow 92 and/or of the produced dihydrogen flow 93 to obtain a primary isotope ratio.

Preferably, the measuring device 30 comprises a communication module 34 suitable to communicate the obtained measurements to the identification device 32.

For example, the measuring device 30 comprises a gas chromatographer, preferably a gas chromatography-isotope ratio-mass spectrometer.

The tagging device 28 and the measuring device 30 may be part of a same device.

The identification device 32 of the third embodiment is similar to the one of the first and second embodiments, and will therefore not be detailed.

The system 9 for verifying the origin of an oxygen-based flow is also similar to the one in the first and second embodiments, and will therefore not be detailed.

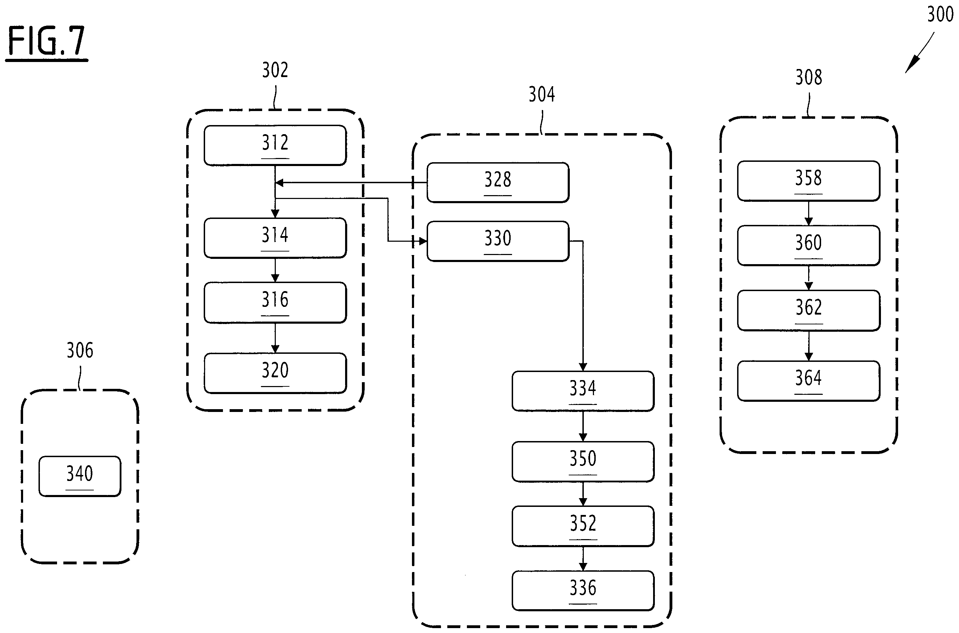

A method for certifying origin of an oxygen-based flow 95, called also method 300, will now be explained in reference to Figure 7 presenting a flowchart of its steps. The method 300 is performed by the system 8 according to the third embodiment of the invention.

The method 300 comprises a production phase 302 and a certification phase 304. Advantageously, the method 300 further comprises a preliminary calibration phase 306 and a final authentication phase 308. Typically, the calibration, the production and the certification phases 306, 302, 304 are carried out by in the producing installation 10 and the authentication phase 308 is carried out in the purchasing installation 12.

The production phase 302 is typically carried out in the producing installation 10 and comprises an initial supplying step 312, a generating step 314 and a collecting step 316 similar to those of the method of the first and second embodiment, the difference being that it is an oxygen-based flow that is produced instead of a hydrogen-based flow.

The certification phase 304 is carried out at least partly simultaneously to the production phase 302.

The certification phase 304 comprises a tagging step 328, a measuring step 330, an associating step 334 and an information storing step 336.

The tagging step 328 and the measuring step 330 may be done simultaneously or successively.

During the tagging step 328, a determined amount of stable ¹⁸O is added in the initial flow 92 or in the produced flow 93

For example, as shown on Figure 6, ¹⁸O-enriched water (H₂¹⁸O) is injected into the water flow 92.

In a variant (not shown), Oxygen-¹⁸O₂ is injected into the produced flow of oxygen.

Alternatively, ¹⁷O-enriched water (H₂¹⁷O) is injected into the water flow 92 or Oxygen-¹⁷O₂ is injected into the produced flow of oxygen.

The measuring step 330 is carried out just after the tagging step 328. During this step, the ¹⁸O/¹⁶O ratio, respectively the ¹⁷O/¹⁶O ratio, is measured by the measuring device 30 in a sample of the tagged flow.

After tagging, the primary isotope ratio ¹⁸O/¹⁶O is typically comprised between 0.2% and 5.0% (molar ratio)., respectively the primary isotope ratio ¹⁷O/¹⁶O is typically comprised between 0.03%t and 1.0% (molar ratio).

The isotope ratio measured during the measuring step 330 is called primary isotope ratio.

Advantageously, the measurements during step 330 are carried out by gas chromatography, notably by gas chromatography-isotope ratio-mass spectrometry.

The rest of the method 300 is similar to the method according to the first embodiment, and will therefore not be further detailed. The same numerical references, only incremented by 200, will be used to designate the same steps.

### OTHER EMBODIMENTS

One can conceive that many other embodiments of the invention are possible. Particularly, it will be clear for a person skilled in the art, that the modules of the systems 8, 9 can be arranged in different way.

According to a variant of the first, second and third embodiments, the comparing step 162, 262, 362 is performed directly in the distant server 49.

According to said variant, the electronic-receiving device 72 generates a request for authenticating comprising the measured ratio and send it to the distant server 49.

Then, the measured ratio is compared with the expected isotope ratio stored in the certificate.

The comparison is done distantly.

If the expected ratio matches the measured ratio, an authentication confirmation is then generated and sent to the electronic-receiving device 72.

If the expected ratio does not match the measured ratio, no authentication confirmation is generated and/or a mismatch signal is then transmitted to the electronic-receiving device 72.

In a variant of the first or second embodiment, the flow of dihydrogen 23 is produced by a process different than water electrolysis during the generation step 114, 214.

For example, the flow of dihydrogen 23 is produced by process chosen among natural gas reforming, methane pyrolysis, biomass pyrolysis, H₂S electrolysis and photoelectrocatalysis

The method performed by the system according to said variant of the invention is similar to the method explained above, except the generating step 114, 214, the first measuring step 130, 230 and the step 140, 142 of acquiring the partition coefficient specific to the production phase 102, 202.

in a variant of the third embodiment, the flow of dioxygen 93 is produced by a process different than water electrolysis during the generation step 114.

For example, the flow of dioxygen 93 is produced by photoelectrocatalysis

According to a variant of the first, second and third embodiments, the certificate is associated with a validity period, the quantity of added isotope being adjusted so that the produced flow of dihydrogen, respectively dihydrogen derivatives or dioxygen, generated during said validity period has the same unique identifier.

Advantageously, the quantity of added isotope is adjusted so that the D/H, respectively the T/H or the ¹⁸O/¹⁶O or ¹⁷O/¹⁶O, ratio equals the unique purchased number.

## Claims

1. A method (100; 200; 300) for certifying origin of a hydrogen-based flow or of an oxygen-based flow, comprising the following steps:
(i) Generating (114; 214, 218; 314), notably by water electrolysis or natural gas reforming, a produced flow of dihydrogen (23) and/or dihydrogen derivatives (25), respectively a produced flow of dioxygen (93), from an initial flow (22; 92);
(ii) Tagging (128; 228; 328) at least one of the initial flow (22; 92) and/or of the produced flow (23; 25; 93) by adding a determined amount of stable isotope chosen between deuterium, tritium,¹⁸O and ¹⁷O;
(iii) Measuring (130; 230; 330) the concentration of said isotope in the initial flow (22; 92) and/or the produced flow (23; 25; 93) to obtain a primary isotope ratio ;
(iv) Associating (134; 234; 334) said primary isotope ratio with a unique identifier to form identification information and storing the identification information in a distant server.

2. The method (100; 200; 300) according to claim 1, further comprising the steps of:
(v) Comparing (162; 262; 362) the identification information with a request for authenticating, said request for authenticating comprising a measured isotope ratio of a hydrogen-based flow (50) to be authenticated, respectively an oxygen-based flow (95) to be authenticated; and
(vi) Based on the comparison, generating (164; 264; 364) an authentication confirmation if the identification information matches the request for authenticating.

3. The method (100; 200; 300) according to claim 2, wherein the measured isotope ratio of the flow to be authenticated is obtained by spectroscopy, preferably by high-resolution mass spectrometry.

4. The method (100; 200; 300) according to claim 2 or 3, further comprising a step of storing (120; 220; 320) and transporting the produced flow prior to the step (162; 262; 362) of comparing the identification information with a request for authenticating.

5. The method (100; 200) according to any of the preceding claims, wherein the produced flow of dihydrogen (23) and/or dihydrogen derivatives (25) is generated by water electrolysis of a water flow, the tagging step (128; 228) comprising :
(iia) injecting heavy water D₂O and/or tritiated water T₂O into the water flow.

6. The method (100; 200) according to any of the preceding claims, wherein the tagging step (128; 228) comprises :
(iib) injecting a gas chosen between D₂ gas and T₂ gas into the produced flow of dihydrogen (23).

7. The method (200) according to any of the preceding claims, wherein the step of generating the produced flow of dihydrogen derivatives (25) comprises a step (214) of generating a flow of dihydrogen (23) and a step (218) of converting said flow of dihydrogen (23) into a produced hydrogen-based flow (25) by chemical reaction with a reacting agent from a reacting flow (24), said produced hydrogen-based flow (25) comprising H₂ derivatives, preferably chosen between NH₃, CH₄ or CH₃OH, or methylcyclohexane

8. The method (300) according to any of claims 1 to 4, wherein the produced flow of oxygen (93) is generated by water electrolysis of a water flow, the tagging step (328) comprising :
(iia) injecting ¹⁸O-enriched water (H₂¹⁸O) into the water flow or injecting ¹⁷O-enriched water (H₂¹⁸O) into the water flow.

9. The method (300) according to any of claims 1 to 4 and 8, wherein the tagging step (328) comprises :
(iib) injecting Oxygen-¹⁸O₂ into the produced flow of oxygen (93) or injecting Oxygen-¹⁷O₂ into the produced flow of oxygen (93).

10. The method (100; 200; 300) according to any one of the preceding claims, wherein the unique identifier is obtained by computing the primary isotope ratio with a partition coefficient specific to the generating step (114; 214; 314)., the unique identifier being preferably defined by an electronic computer, this electronic computer comprising a model having in memory the partition coefficient specific to the generating step.

11. The method (100; 200; 300) according to any of the preceding claims, further comprising a step (152; 252; 352) of emitting a certificate comprising the identification information and sending said certificate to an electronic receiving device (72).

12. The method according to claim 11, wherein the certificate is an electronic certificate comprising a unique token, said token being associated to a purchased number ranging from 0 to 1 (excluded) corresponding to the unique identifier, the step of associating the token to a purchased number being preferably done by an algorithm.

13. The method according to any of claim 11 and 12, wherein the certificate is associated with a validity period, the quantity of added isotope being adjusted so that the produced flow of dihydrogen (23) and/or dihydrogen derivatives (25), respectively of the produced flow of dioxygen (93), generated during said validity period has the same unique identifier.

14. A computer program product comprising software instructions which, when executed by a processor, implement a method (100; 200; 300) according to any of the preceding claims.

15. A system (8) for certifying origin of a hydrogen-based flow (23; 25) and/or of an oxygen-based flow (93), the system being configured to carry out the method (100; 200; 300) according to any one of claims 1 to 13, the system comprising :
- a tagging device (28) for tagging at least one of the initial flow (22; 92) and/or of the produced flow (23; 25; 93) by adding a determined amount of isotope;
- a measuring device (30) for measuring the concentration of said isotope in the initial flow and/or the produced flow to obtain an primary isotope ratio ;
- an identification device (32) for associating said primary isotope ratio with a unique identifier to form identification information and storing the identification information in a distant server (49).
